Europäisches Patentamt

European Patent Office

Office européen des brevets

(19)

(11) Publication number: **0 101 650**
**B1**

(12) **EUROPEAN PATENT SPECIFICATION**

(45) Date of publication of patent specification: **14.01.87**

(21) Application number: **83304777.2**

(22) Date of filing: **18.08.83**

(51) Int. Cl.⁴: **D 01 F 6/06,** D 01 F 1/10,
A 61 L 15/00

(54) **Radio opaque fibres.**

(30) Priority: **20.08.82 GB 8224055**

(43) Date of publication of application:
**29.02.84 Bulletin 84/09**

(45) Publication of the grant of the patent:
**14.01.87 Bulletin 87/03**

(84) Designated Contracting States:
**AT BE CH DE FR GB IT LI LU NL SE**

(56) References cited:
**DE-A-1 936 976**

**POLYMER-PLASTICS TECHNOLOGY AND
ENGINEERING, vol. 1, no. 1, 1981, pages
95-122, New York, USA, S.J. MONTE et al.:
"United States and Japanese application of
titanate coupling agents in polyolefins"**

(73) Proprietor: **VERNON-CARUS LIMITED
Hoddlesden Mills
Darwen, BB3 3NW Lancashire (GB)**

(72) Inventor: **Carus, Edmund Hugh
14 Carus Avenue
Hoddlesden Darwen Lancashire (GB)**
Inventor: **White, Eric Frank Thomas
Smallridge Mottram Road
Alderley Edge Cheshire (GB)**

(74) Representative: **Funge, Harry et al
WILSON, GUNN & ELLIS 41-51 Royal Exchange
Cross Street
Manchester M2 7BD (GB)**

## Description

This invention relates to fibres, yarns or threads which include radio opaque material.

Radio opaque multifilament yarns are included in surgical swabs so that they can be identified by X-rays. Such yarns must contain at least 55% by weight of radio opaque material in order to meet the specification of the British Pharmacopoeia. In the past, radio opaque yarns have been produced from viscose loaded with up to 60% barium sulphate. However these yarns possessed several shortcomings. The viscose component was affected by wet processing of the yarn during incorporation into woven gauze. Length changes occurred and excessive tensions were apparent. The viscose component was subject to brittle fracture at low stress leading to fragmentation. The nature of the wet spinning process made it very difficult to maintain product consistency.

Polypropylene has been assessed as an alternative carrying medium to viscose. Polypropylene being hydrophobic is not subject to dimensional changes caused by wet processing. Barium loaded polypropylene yarns have been made. However at loadings in excess of 45% by weight extensive loss of strength has been observed in multifilament yarns. Extrusion of filaments at loadings in excess of 45% has been difficult due to barium sulphate agglomeration. Twisting the low strength filaments also poses many difficulties.

We have now discovered that if a coupling agent is applied between the barium sulphate and the polypropylene as a monomolecular layer on the filler, yarns of higher intrinsic strength with enhanced spinnability can be obtained. Titanate coupling agents have been disclosed by S. S. Monte & G. Sugerman (Poly.-Plast. Technol. Eng., 17(1), 95—122 (1981)).

According to a first aspect of the present invention there is provided a radio opaque fibre comprising a mixture of polypropylene, barium sulphate and a coupling agent comprising isopropyl triisostearoyl titanate characterised in that the fibre is a multifilament fibre containing 55% to 70% by weight of barium sulphate and in that the mixture has a melt flow index not less than 12.

There is preferably 60% to 70% of barium sulphate present, more preferably 65% to 70% of barium sulphate.

The titanate coupling agent may be used in an amount of 0.5% to 5% by weight of barium sulphate, an amount of 0.4% to 1% being preferred.

The fibre may incorporate a dye, in accordance with medical practice. For example Cobalt Blue (Med) may be employed.

Multifilament fibre in accordance with the invention may have a twist factor of 4 to 5 turns per inch (1.6 to 2 turns per cm).

Multifilament fibre in accordance with the invention may have a thickness equivalent to 3300 to 3500 decitex (0.33 to 0.35 gm$^{-1}$).

A method of preparation of radio opaque fibre as hereinbefore disclosed comprises combining powdered barium sulphate with the coupling agent, mixing with the polypropylene and extruding the mixture.

The polypropylene is preferably powdered although chips may also be used.

The barium sulphate preferably has a particle size of not greater than 0.5 μ.

The extrusion is preferably carried out under conditions normally used for extruding pure polypropylene with a melt flow index which is not less than 12. However the spinning temperature may be lowered to prevent formation of bubbles in the fibres. Preferred spinning temperatures lie in the range 190—210°C.

Extrusion in a vertical direction is preferred to minimise breakage of the fibre. A drawdown length in excess of 1 m may be employed.

Spin finishing of the fibre is not essential but treatment with a smooth amount of liquid paraffin can be beneficial.

The following Examples further illustrate the invention.

Example 1

Barium sulphate (60 parts, w/w, particle size of 0.5 μ) was weighed and charged into a high shear mixer. Isopropyl triisostearoyl titanate (0.6 parts, w/w was blended with liquid paraffin BP (0.6 parts, w/w) to enhance subsequent distribution of the titanate. The titanate mixture was poured or sprayed into the vortex of barium sulphate as the mixer was operated at high speed. Mixing was continued for three minutes. The mixture was fed into an extruder with polypropylene chips (40 parts, w/w melt flow index 12) to form suitable pellets. A yarn was then extruded from these pellets in a vertical direction. A drawdown length of greater than 1 m was employed. The melt flow index of the compounded polypropylene would not generally exceed 20.

Example 2

Example 1 was repeated with the paraffin oil replaced by toluene (0.6 parts, w/w). Weakening of the filaments by the oil were therefore obviated.

Example 3

Example 1 was repeated using barium sulphate (70 parts, w/w, 0.5 μ), isopropyl triisostearoyl titanate (0.5 parts, w/w) and powdered polypropylene (29.5 parts w/w) melt flow index 12. A fibre was spun at a temperature between 190 and 200°C. The resultant fibre had a twist factor of between 1.6 and 2 turns per cm and a thickness equivalent to 0.33 to 0.35 g · m$^{-1}$. The melt flow index of the compounded polypropylene would not generally exceed 20.

## Claims

1. A radio opaque fibre comprising a mixture of polypropylene, barium sulphate and a coupling agent comprising isopropyl triisostearoyl titanate characterised in that the fibre is a multifilament fibre containing 55% to 70% by weight of barium sulphate and in that the mixture has a melt flow index not less than 12.

2. A radio opaque fibre as claimed in claim 1, characterised in that the fibre contains 60% to 70% by weight of barium sulphate.

3. A radio opaque fibre as claimed in claim 2, characterised in that the fibre contains 65% to 70% by weight of barium sulphate.

4. A radio opaque fibre as claimed in any preceding claim, characterised in that the fibre has a twist factor of 1.6 to 2 turns per centimetre.

5. A fibre as claimed in any preceding claim, characterised in that the thickness is equivalent to 0.33 to 0.35 gm$^{-1}$.

6. A fibre as claimed in any preceding claim, characterised in that the fibre is spun at a temperature of 190—210°C.

## Patentansprüche

1. Strahlenundurchlässige Faser, welche eine Mischung aus Polypropylen, Bariumsulfat und einem Haftmittel umfasst, wobei das Haftmittel seinerseits Isopropyl-Triisostearyl-Titanat umfasst, dadurch gekennzeichnet, daß sie eine mehrfädige Faser ist, welche zwischen 55 und 70 Gewichtsprozent Bariumsulfat enthält, und daß die Mischung einen Schmelzindex aufweist, der nicht kleiner als 12 ist.

2. Strahlenundurchlässige Faser nach Anspruch 1, dadurch gekennzeichnet, daß sie zwischen 60 und 70% Bariumsulfat enthält.

3. Strahlenundurchlässige Faser nach Anspruch 2, dadurch gekennzeichnet, daß sie zwischen 65 und 70 Gewichtsprozent Bariumsulfat enthält.

4. Strahlenundurchlässige Faser nach einem der vorhergehenden Ansprüche, dadurch gekennzeichnet, daß sie einen Twistfaktor von 1,6—2 Umdrehungen pro Zentimeter aufweist.

5. Faser nach einem der vorhergehenden Ansprüche, dadurch gekennzeichnet, daß die Dicke 0,33—0,35 gm$^{-1}$ entspricht.

6. Faser nach einem der vorhergehenden Ansprüche, dadurch gekennzeichnet, daß sie bei einer Temperatur von 190—210° Celsius gesponnen ist.

## Revendications

1. Fibre opaque à la radio comprenant un mélange de polypropylène, sulfate de baryum et un agent de couplage comprenant du titanate d'isopropyltriisostéaryle, caractérisée par le fait que la fibre est une fibre multifilament contenant 55% à 70% en poids de sulfate de baryum et que le mélange à un indice d'écoulement à l'état fondu non inférieur à 12.

2. Fibre opaque à la radio selon la revendication 1, caractérisée par le fait que la fibre contient 60% à 70% en poids de sulfate de baryum.

3. Fibre opaque à la radio selon la revendication 1 ou 2, caractérisée par le fait que la fibre contient 65 à 70% en poids de sulfate de baryum.

4. Fibre opaque à la radio selon l'une des revendications précédentes, caractérisée par le fait que la fibre possède un facteur de torsion de 1,6 à 2 tours par centimètre.

5. Fibre selon l'une des revendications précédentes, caractérisée par le fait que l'épaisseur est équivalente à 0,33 à 0,35 gm$^{-1}$.

6. Fibre selon l'une des revendications précédentes, caractérisée par le fait que la fibre est filée à une température de 190—210°C.